# EUROPEAN PATENT SPECIFICATION

(11) **EP 0 699 235 B1**
(45) Date of publication and mention of the grant of the patent: **09.05.2007**
(21) Application number: 94917988.1
(22) Date of filing: 13.05.1994
(51) Int. Cl.: C12N 15/00

(54) **ANIMAL MODEL FOR HEPATITIS VIRUS INFECTION**
TIERMODELL FÜR HEPATITIS-VIRUS INFEKTION
MODELE ANIMAL POUR L'INFECTION PAR LE VIRUS DE L'HEPATITE

(30) Priority: 17.05.1993 US 61706; 08.09.1993 IL 10695193
(43) Date of publication of application: 06.03.1996
(73) Proprietor: YEDA RESEARCH AND DEVELOPMENT COMPANY, LTD., 76 100 Rehovot (IL)
(72) Inventor: REISNER, Yair, Tel Aviv (IL)
(74) Representative: Vossius & Partner
(86) International application number: PCT/US1994/005410
(87) International publication number: WO 1994/027556

(56) References cited:
- EP-A- 0 438 053
- EP-A- 0 469 632
- EP-A- 0 517 199
- SCIENCE, vol.252, 19 April 1991 pages 427 - 431 LUBIN, I. ET AL. 'Engraftment and development of human T and B cells in mice after bone marrow transplantation'
- PROC.NATL.ACAD.SCI., vol.90, July 1993, USA pages 6037 - 6041 SHIMIZU, Y. ET AL. 'Correlation between the infectivity of hepatitis C virus in vivo and its infectivity in vitro' cited in the application
- PROC.NATL.ACAD.SCI., vol.83, June 1986, USA pages 4529 - 4532 NAKAMURA, T. ET AL. 'Successful liver allografts in mice by combination with allogeneic bone marrow transplantation' cited in the application
- ADVANCES IN IMMUNOLOGY, vol.50, 1991 pages 303 - 325 MOSIER, D.E. 'Adoptive Transfer of Human Lymphoid cells to severely immunodeficient mice: models for normal human immune function, autoimmunity, lymphomagenesis and AIDS'
- HEPATOLOGY, vol.20, no.4/2, 1994 page 232A GALUN, E. ET AL. 'Hepatitis C viremia in chimeric mice'
- BLOOD, vol.83, no.8, 1994 pages 2368 - 2381 LUBIN, I. ET AL. 'Engraftment of human peripheral blood lymphocytes in normal strains of mice'

## Description

### FIELD OF INVENTION

The present invention concerns an animal model for hepatitis virus (HV) infection in humans, particularly hepatitis B virus (HBV) and hepatitis C virus (HCV) infection.

### PRIOR ART

The following is a list of prior art and references considered to be pertinent for the description below:
1. Choo, Q-L, Kuo G, Weiner, A.J. Overby L.R., Bradley D.W., Houghton, M. Isolation of cDNA clone derived from a blood-borne non-A, non-B viral hepatitis genome. 1989. *Science* **244**:359-362.
2. Kuo G, Choo Q-L, Alter H.J., Gitnick, G.L., Redeker A.G., Purcell R.H., Miyamura T, Dienstag J.L., Alter H.J. Stevenes C.E., Tegtmeier G.E., Bonnino F., Colombo M, Lee W-S, Kuo C, Berger K, Shuster J.R., Overby L.R., Bradley D.W., Houghton M. 1989. An essay for circulating antibodies to major etiologic virus of human non-A, non-B hepatitis. *Science* **244**:362-344.
3. Prince, A.M., Brotman B., Huima T., Pascual D., Jaffery M. Inchauspe G. 1992. Immunity in hepatitis C infection. *J. Infec. Dis.* **165**:438-443.
4. Shimizu Y.K., Weiner, .J. Rosenblatt J., Wong D.C., Shapiro M., Popkin T, Houghton M, Alter H.J., Purcell R.H. 1990. Early events in hepatitis C virus infection in chimpanzees. *Proc. Natl. Acad. Sci.* (USA) **87**:6441-6444.
5. Shimizu, Y.K., Iwamoto A., Hijikata M., Purcell R.H., Yoshikura H. 1992. Evidence for in vitro replication of hepatitis C virus genome in a human T cell line. *Proc. Natl. Acad. Sci.* (USA) **89**:5477-5481.
6. Shimizu Y.K., Purcell R.H., Yoshikura H. 1993. Correlation between the infectivity of hepatitis C virus *in vivo* and its infectivity *in vitro. Proc. Natl. Acad. Sci.* (USA) **90**:6037-6041.
7. Nakamura T., Good R.A., Yasumizu R., Inoue S., Oo M.M., Hamashima Y, Ikehara S. 1986. Successful liver allografts in mice by combination with allogeneic bone marrow transplantation. *Proc. Natl. Acad. Sci.* (USA) **83**:4529-45326.
8. Bosma, M.J. Carroll, A.M. 1991. The SCID mouse mutant: Definition, characterization, and potential uses. *Annu. Rev. Immunol.* **9**:323-350.
9. Soriano, H.E., Adams, R.M., Darlington G., Finegold M. Steffen D.L., Ledley F.D. 1992. Retroviral transduction of human hepatocytes and orthotopic engraftment in SCID mice after hepatocellular transplantation. *Trans. Proc.* **24**:3020-3021.
10. Aldrovandi G.M., Feurer G., Gao L., Jamieson B., Kristeva M., Chen I.S.Y., Zack J.A., 1993. The SCID-hu mouse as a model for HIV-1 infection. *Nature* **363**:732-736.
11. European laid open Patent Application, Publication No. 438053.
12. European laid open Patent Application, Publication No. 517199.

The citation herein of the above publications is given to allow an appreciation of the prior art. This citation should not, however, be construed as an indication that this art is in any way relevant to the patentability of the invention, as defined in the appended claims.

The above publications will be acknowledged herein by indicating their number from the above list.

### BACKGROUND OF THE INVENTION

Five different viruses have been identified as causes of viral hepatitis. These include hepatitis A, B, C, D and E viruses. Of these, the viruses which cause the most serious infections are hepatitis B virus (HBV) and hepatitis C virus (HCV).

Hepatitis A virus has a single serotype and causes a self-limited acute infection. A large percentage of the population, approaching 50%, has hepatitis A antibodies in serum and is probably immune to disease. Infection with hepatitis A does not progress to chronic disease.

HBV is implicated in both acute and chronic hepatitis. The disease is endemic in Asia, is increasing in prevalence in the U.S. and Europe. Chronic liver disease, resulting in significant morbidity and increased mortality, is sequela of infection in 1-10% of infected individuals. HBV infection is also correlated with the development of primary liver cancer.

HCV was recently shown to be the major causative agent of parenterally transmitted non-A, non-B hepatitis⁽¹⁾. It is estimated that 0.5-1% of the world population is infected with HCV, and in some developing countries the prevalence rate is up to 40%. Moreover, 40-60% of newly infected patients develop persistent HCV infections⁽²⁾ and are at risk of developing acute, fulminant hepatitis and various chronic liver diseases (including cirrhosis, chronic active hepatitis and in some cases hepatocellular carcinoma).

Hepatitis D virus (*"Delta Virus"*) is a defective RNA virus that can only infect the liver in the presence of an active HBV infection. Hepatitis E virus appears to be a single-stranded RNA virus. Infection with hepatitis E virus is not known to progress to chronic liver disease.

Although HBV and HCV have been identified and characterized, the development of new anti-viral strategies has been greatly hampered by the lack of adequate, simple and low cost animal model systems.

Currently, biological assays for HBV and HCV have been limited to the experimental inoculation of chimpanzees^{(3,4)}, which are expensive and limited in numbers. In addition, an *in vitro* system for the propagation of HCV was developed in the murine retrovirus infected human T cell lines, HPB-Ma⁽⁵⁾ and Molt4-Ma⁽⁶⁾, in which replication of HCV is achieved.

It has recently been demonstrated in several studies that human solid organs such as fetal thymus or fetal liver as well as several types of tumors were successfully grafted into SCID mice under the kidney capsule⁽⁷⁾. In addition, transplantation of other organs such as lymph nodes and bone marrow spicules and engraftment of organs to other sites (i.e. subcutane and peritoneum) have also been reported.

A SCID mouse mutant was reported to support human cell implantation, i.e. single hepatocyte transplantation^{(8,9)}, and was also used as a model for human infectious diseases, i.e. HIV-1 infection⁽¹⁰⁾.

It has been disclosed that lethally irradiated mice, radio-protected with bone marrow from SCID mice, developed marked immune-deficiency and supported engraftment of human peripheral blood lymphocytes (PBL) for a long period of time⁽¹¹⁾. It was also disclosed that human implants of non-hematopoietic origin were accepted and maintained for prolonged periods of time after transplantation under the kidney capsules of these chimeras⁽¹²⁾.

### GENERAL DESCRIPTION OF THE INVENTION

It is an object of the present invention to provide a convenient non-human animal model for HV infection.

It is further an object of the present invention to provide a method for evaluation of preventive and therapeutic agents for the treatment and prophylaxis of HV infections using the above non-human animal model.

It is still another object of the present invention to provide methods for production of anti-HV xenogeneic antibodies or T cells, and particularly human monoclonal antibodies and cytotoxic T cells, using chimeric non-human mammals transplanted with human hematopoietic cells and human liver tissue infected by HV either pre- or post-transplant.

The present invention provides, by its first aspect, a non-human chimeric animal useful as a model for human HV infection, comprising a non-human mammal M5 having xenogeneic cells; mammal M5 being derived from a non-human mammal M1 treated to substantially destroy its hematopoietic cells and then transplanted with hematopoietic cells derived from one or more mammals M2 and transplanted with HBV- or HCV-infected liver tissue from a mammal M3, the one or more mammals M2 and mammal M3 being from the same or from a different species; the transplanted hematopoietic cells from the one or more mammals M2 being either one or both of a hematopoietic cell preparation from a T cell deficient mammal or of a T cell depleted mammalian stem cell or bone marrow preparation; the transplanted liver tissue from mammal M3 being either a human liver tissue preparation or a liver tissue preparation from a non-human mammal capable of being infected by HV.

The non-human M1 mammal may typically be a mouse or a rat, although the M1 mammal may also be a non-human mammal of a higher order such as a primate, e.g. marmoset monkeys.

For the obtaining of a non-human M5 mammal from said non-human M1 mammal, the M1 mammal is first treated in a manner so as to substantially destroy its hematopoietic system. The term *"substantially destroyed"* should be understood as meaning that the number of hematopoetic cells which survive following the treatment are insufficient to immune-protect the animal in the absence of the transplant from the M2 mammal. Following treatment intended to substantially destroy the hematopoietic cells, some such cells survive but the number is small such that the animal could not survive under normal laboratory conditions.

A treatment intended to substantially destroy the hematopoietic cells may, for example, be a split dose total body irradiation, (TBI). A TBI effective in destroying the hematopoietic system requires typically an accumulative dosage of 4-50 Gy (1 Gy = 100 rad). In the case of a mouse, the irradiation may, for example, be a 4 Gy on day 1 and 9-15 Gy three days later. A similar irradiation dose was found to be effective in destroying the hematopoietic cells also in rats and marmoset monkeys.

The M2 mammal may be from the same or a different species than the M1 mammal. In principle, any mammal with a T cell deficiency may serve as a donor for the transplanted hematopoietic cells. An example of an M2 donor is a severe combined immuno-deficient (SCID) mouse or a SCID animal from another mammalian species or genera. The transplanted hematopoietic cell preparation in this case is suitably a bone marrow preparation.

The transplanted hematopoietic cells derived from the M2 mammal may also be a T cell depleted hematopoietic stem cell preparation obtained from a donor M2 mammal, such as a primate, e.g. a monkey or a human. In the case of humans, a stem cell enriched preparation may, for example, be obtained from peripheral blood of donors pretreated with a granulocyte colony stimulating factor (G-CSF) or from cancer patients undergoing chemotherapy known to cause migration of stem cells to the periphery. After withdrawal of the blood preparation from such donors, the preparation is typically treated to remove various blood components and to deplete the T cells therefrom. For T cell depletion, the M2 derived hematopoietic cell preparation may be subjected to treatment intended for enrichment with cells displaying the CD34 antigen (CD34⁺ cells). Each of the above stem cell enriched, T cell depleted preparations can either be used directly after their withdrawal from the donor, or may be a cell-preparation which underwent one or a plurality of passages *in vitro.*

The transplanted hematopoietic cells may also be a T cell depleted bone marrow preparation.

The M1 mammal may also be transplanted with both a hematopoietic cell preparation from a T cell deficient mammal and a T cell depleted mammalian stem preparation. A specific example is a combined transplantation of bone marrow from a SCID mammal, e.g. a SCID mouse, and a T cell depleted human bone marrow preparation.

In order to obtain the M5 mammal, the M1 mammal may be transplanted with an HV infected liver tissue. Such an HV infected liver tissue may be obtained from an M3 mammal infected with HV, e.g. a liver biopsy from an HV infected human. Furthermore, an HV infected liver tissue preparation may also be obtained by in *vitro* infection of an *a priori* non-HV infected liver tissue preparation obtained from a non-HV infected M3 donor mammal.

In addition to human liver tissue preparation, it is also possible to use liver tissue preparations from non-human M3 mammals susceptible to HV infections such as chimpanzees or other non-human primates.

The animal model of the invention is particularly suitable for the study of the pathology for HBV and HCV infections and the development of therapies therefor. Models for both HBV and HCV are particularly preferred in accordance with the invention, as no simple and low cost models for these viral infections are currently available.

The invention further provides, by a second of its aspects, a method for evaluating the potential of an agent or a combination of agents in the therapy of an HV infection using a non-human M5 mammal as defined above; said method comprising:
(a) administering said agent or said combination of agents to said M5 mammal; and
(b) evaluating the effectiveness of said agent or said combination of agents in preventing spread of HV infection, reducing its physiological symptoms or reducing the evidence of active infection in said M5 mammal.

By a modification thereof, the above method may also be applied in determining the effective dose of said agent or said combination of agents in therapy or prevention, as the case may be.

By a further aspect, the present invention provides a method of obtaining anti-HV immune cells or antibodies using a non-human mammal M5, as defined above, wherein at least one of said one or more M2 mammals is human; said method comprising:
(a) recovering immune cells or antibodies from the blood of said M5 mammal; and
(b) selecting for the immune cells or antibodies having an anti-HV reactivity.

Optionally, in accordance with this aspect, the M5 mammal, is treated so as to increase the immune response against HV, such as for example by vaccination.

The selected immune cells may be cytotoxic T cells reactive against HV infected liver cells. Such a cytotoxic T cell preparation may be obtained by growing lines of T cells obtained from the M5 mammal and then selecting those lines which develop a cytotoxic cell response against cells expressing HV antigens. Such a cytotoxic T cell preparation may be injected to HV patients within the framework of an anti-HV therapy.

The selected immune cells may also be antibody producing B cells immortalized and selected for those producing anti-HV antibodies. The antibodies produced by these B cells may then be used as therapeutic agents in anti-HV therapies of hepatitis patients.

The manner of growing cytotoxic T cell lines, the manner of immortalization of B cells to produce B cell lines, as well as the manner of selecting specific cytotoxic T cells or immortalized B cell lines to obtain those having the desired reactivity, is generally known *per se* and the full explanation of such methods goes beyond the present writing.

The invention will now be described with reference to some specific embodiments described in the following examples and the appended drawings.

### DESCRIPTION OF THE DRAWINGS

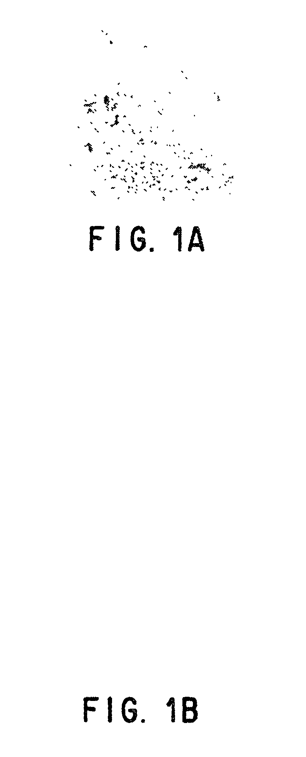

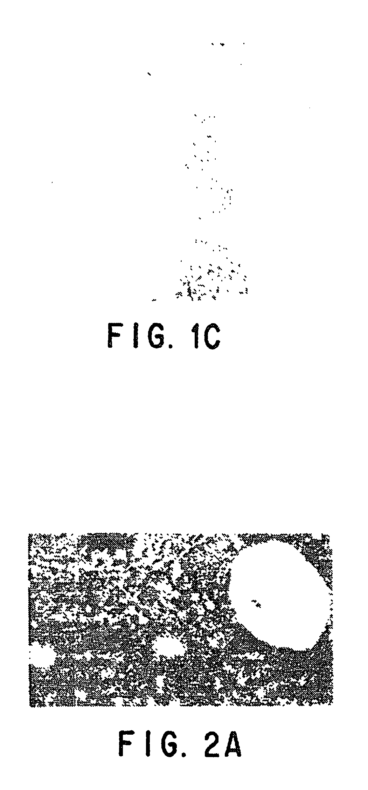

**Fig. 1** shows histology of liver tissue transplanted under the kidney capsule of SCID-BNX chimera, 1 month after transplantation of liver fragments from BNX mouse (A), Lewis rat (B), or human (C). All transplanted animals which survived the kidney subcapsular implantation of liver tissue were assessed for engraftment by light microscopy of the kidneys using H&E staining.

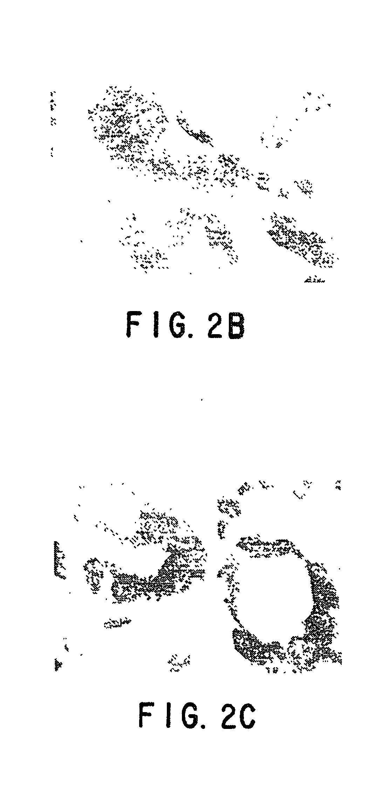

**Fig. 2** shows hepatocytes and bile duct-like structures (marked with arrows) in a transplanted human liver fragment 30 days after grafting at x240 magnification (A); hepatocytes and epithelial cells at x1200 magnification (B), bile duct-like structures at x1200 magnification (C).

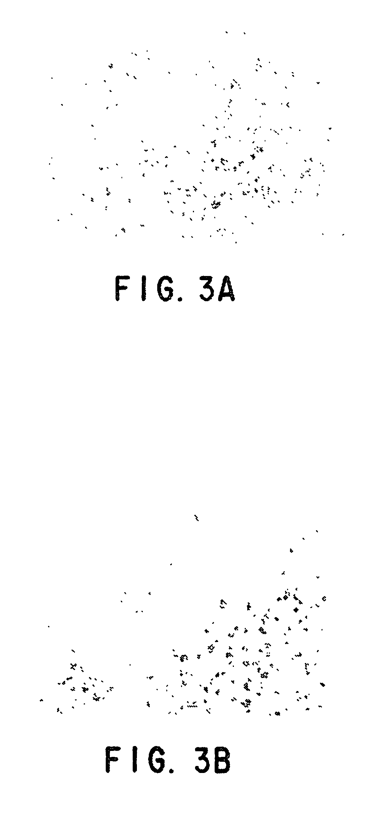

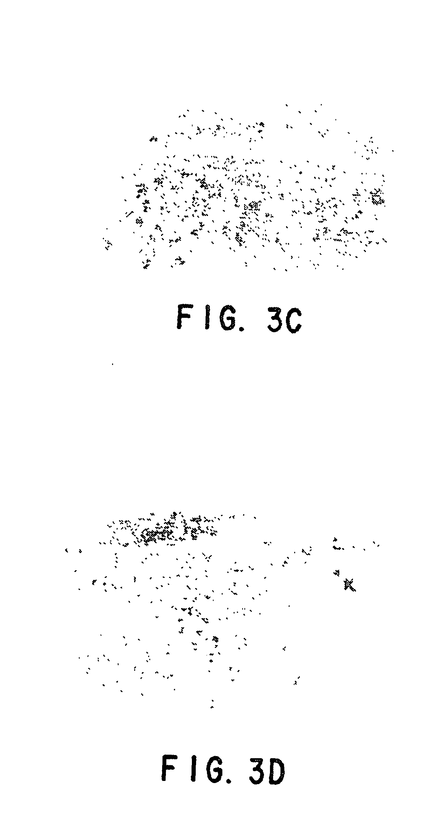

**Fig. 3** shows (A) histology of a liver segment prior to transplantation (H&E, x100) (B), immunohistological staining for HBsAg of human liver, infected with HBV (x100) (C), periodic acid-Schiff staining for glycogen of a human liver (L) segment transplanted under the kidney (K) capsule of a SCID-BNX chimera (x50) and (D), immunohistological staining for HBsAg of human liver infected with HBV, as observed 19 days after transplantation at the subcapsular area of the SCID-BNX chimera kidney (x50). Staining was performed using, as a primary antibody, mouse anti-HBsAg (Zymed Lab, San Francisco, CA).

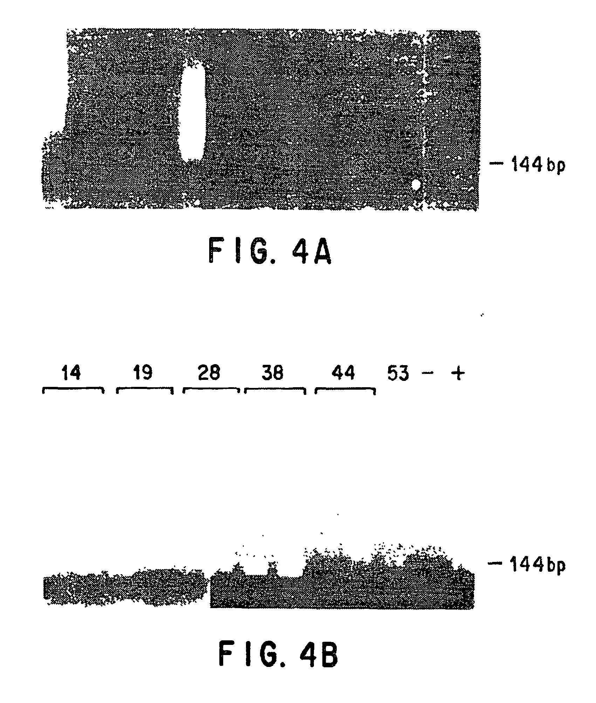

**Fig. 4** is an electrophoretogram of cDNA of RNA samples extracted from sera of SCID-BNX chimera, transplanted under the kidney capsule with human liver infected with HCV, and subjected to RT-PCR amplification using two sets of primers as described in Example 3. Each lane represents a different mouse which was sacrificed at the day of sampling. The first 13 lanes, which are shown at the upper part of Fig. 4, are from mice at day 9 after transplantation. The remaining lanes are from days 14-53 after transplantation (the days are indicated above the lanes). The last two lanes are negative control (-) and positive control (+).

### EXAMPLES

### Example 1: Engraftment of Human Liver Segment from non-HCV Patients

BNX mice (6-10 weeks old, female) were purchased from Harlam Sprague-Dawley (Indianapolis, IN) and CB17/SCID mice were from the Animal Breeding Center, Weizmann Institute, Rehovot, Israel. Mice were kept in small cages (5 animals in each cage) and fed sterile food and acid water containing cyprofloxacin (20 mg/ml). Prior to transplantation, the BNX mice were conditioned with 12 Gy TBI and radioprotected the following day with 2-3x10⁶ T cell depleted SCID bone marrow cells. TBI was administered from a gamma beam 150-A ⁶⁰Co source (Atomic Energy of Canada, Kanata, Ontario) with F.S.D. of 75 cm and a dose rate of 0.7 Cy/min. Bone marrow cells obtained from SCID mice (4-10 weeks old) were fractionated by differential agglutination with soybean agglutinin (to remove T lymphocytes that may be present in occasional "leaky" SCID mice) as previously described (11). One day after bone marrow transplantation, human, rat or mouse liver fragments were grafted under the kidney capsule.

Rat and mouse liver tissue fragments were collected through laparotomy, in which a wedge biopsy was cut from the animal liver and kept under sterile conditions at 4°C, in Dulbecco modified Eagle medium containing 10% fetal calf serum or ViaSpan (Belzer UW solution, Du Pont Pharmaceuticals, Hertogenbosch, The Netherlands).

Human liver segments were obtained during hepatic segmentectomy when performed for primary or secondary liver tumors. In all cases, the non-tumor tissue was non-cirrhotic, as confirmed by hematoxylin and eosin (H&E) staining. The liver segments were kept, for up to 2 hours in UW solution prior to transplantation. For engraftment of liver tissue, BNX mice were anesthetized with Nembutol or Avertin. An incision of approx. 1 cm was then made in the right or left flank, the kidney was exposed and liver tissue (cut into 1 mm² pieces) was placed under the renal capsule using fine forceps. One suture was placed to close the wound. Kidneys, with the attached transplanted tissue, were removed at various time intervals (from 8 days to 3 months), fixed in Bouin's liquid, embedded in paraffin, and 4 µm sections were stained with H&E.

A summary of the transplantations of liver fragments in the SCID-BNX chimeric mice is shown in the following Table 1.

**Table 1**

| **Number of Mice** | | | **Source of liver Follow-up** | |
|---|---|---|---|---|
| **Transplanted** | **Surviving** | **Engrafted** | (weeks) | |
| 10 | 9 | 4 | Mouse | 10 |
| 20 | 12 | 8 | Mouse | 3 |
| 10 | 7 | 4 | Mouse | 1.4 |
| 10 | 10 | 8 | Rat | 10 |
| 20 | 8 | 5 | Rat | 8 |
| 20 | 7 | 2 | Rat | 2 |
| 10 | 5 | 2 | Rat | 1.4 |
| 20 | 11 | 5 | Human | 14 |
| 20 | 11 | 5 | Human | 14 |
| 20 | 11 | 5 | Human | 12 |
| 20 | 10 | 2 | Human | 6 |
| 20 | 9 | 3 | Human | 4 |
| 20 | 16 | 12 | Human | 2 |
| 20 | 15 | 14 | Human | 2 |
| 16 | 12 | 9 | Human | 2 |

As seen from the above table, the survival rate of the chimeric mice receiving the human liver graft was at the order of about 50-60%.

Histological examination of the transplanted liver fragments at this subcapsular area of the transplanted SCID-BNX chimera showed that following transplantation the typical liver cell architecture disappeared and in most transplants, central ischemia occurred while the peripheral tissue of the transplant was markedly fibrotic (see Fig. 1). In most cases, hepatocytes were recognized in addition to proliferating epithelial cells (Fig. 2) and the engrafted tissue mainly resembled the morphological characteristics of biliary epithelium (Fig. 2B and 2C), A very mild inflammatory reaction was occasionally observed consisting a few polymorphonuclear and plasma cells.

Evaluation of liver engraftment rate showed that among the mice receiving the human liver grafts, 15 out of 33 retained the graft for more than 12 weeks while 40 out of 62 were stably engrafted at six weeks or less (Table 1).

### Example 2: Transplantation of Liver Tissue from a human infected with hepatitis B virus (HBV)

A liver biopsy from a patient infected with hepatitis B virus (HBV) was obtained and transplanted under the kidney capsule of SCID/BNX chimera mice as described in Example 1. Immunohistology of the liver segment prior to transplantation showed that the hepatocytes stained positively for the HBV surface protein (HBsAg) (Fig. 3B). After transplantation, evidence was provided for the human origin of the transplanted cells by using staining with periodic acid-Schiff reaction which identifies glycogen in human hepatocytes (Fig. 3C). 19 days after transplantation, the subcapsular area of the SCID-BNX chimera kidney was immunohistologically stained and HBsAg was detected in discrete areas of the cytoplasm in the engrafted tissue but not in the neighboring mouse kidney cells (Fig. 3D).

### Example 3: Transplantation of Liver Fragments Infected in vitro with HBV

Liver fragments were obtained from non HBV infected humans as described in Example 1. The non infected human liver fragments were incubated in *vitro* with HBV resulting in their infection by HBV. The in *vitro* HBV infected liver fragments were transplanted under the kidney capsule of SCID-BNX mice as described in Example 1 and the detection of HBV in the transplanted mice was assessed either by immunohistology of the hepatocytes as described in Example 2 or by testing the level of HEV in the serum of the mice by PCR reaction (RT-PCR) as follows:

The results are shown in the following Table 2:
DNA was dissolved in 30 µl DNAse-free H₂O. PCR was performed in reactions mixture volume of 100 µl containing Taq Polymerase Buffer (Promega) 2 mM dNTP, 1.5 mM MgCL₂, 500 ng each of sense (Bs) and antisense (Bas) primers, and 2.5 units Taq Polymerase (Promega). The reaction was carried out by 35 cycles of PCR consisting of 94°C for 1.5 min, 55°C for 1.5 min and 72°C for 3 min.
The following primers used:
Bs: 1778-1806 5'-GGA-GGC-TGT-AGG-CAT-AAA-TTG-GTC-TGC-GC-
Bas: 2446-2408 5'-CCC-GAG-ATT-GAG-ATC-TTC-TGC-GAC-GCG-GCG-ATT-GAG-ACC-

**Table 2 - Transplantation Results of in vitro Viral Hepatitis B Infected Human Livers in SCID-BNX Mice**

| **No. of Animals** | **No. of Animals Survived** | **No. of Animals Engrafted** | **Route of Infection*** | **Infection Indicators and comments** |
|---|---|---|---|---|
| 10 | 5 | 5 | Pre-transplantation *in vitro* incubation of liver fragments with HBV-DNA, +ve sera | PCR +ve (3/5) |
| 10 | 7 | ND*** | Pre-transplantation *in vitro* incubation of liver fragments with HBV-DNA, +ve sera | PCR +ve 1/7 at day 11 PCR +ve 4/7 at day 30 |
| 10 | 5 | ND | Pre-transplantation *in vitro* incubation of liver fragments with HBV-DNA, +ve sera | PCR +ve 1/5 at day 11 |

| | | | | |
|---|---|---|---|---|
| * +ve sera = sera positive for viremia ** PCR +ve = PCR test positive for hepatitis virus DNA *** ND = No Data: Mice have not yet been sacrificed for histological examination. | | | | |

As seen in the above table, HBV sequences were observed in the sera of some of the transplanted mice about ten days after transplantation and with progression of time after transplantation HBV sequences were observed in a larger number of transplanted mice.

### Example 4: Detection of HCV in sera of mice transplanted with liver fragments from HCV infected patients

Liver fragments from three patients with chronic HCV infection were obtained and transplanted under the kidney capsule of SCID-BNX mice as described in Example 1. The presence and level of HCV in the serum of the transplanted mice was assessed by reverse transcriptase-nested-polymerase chain reaction (RT-PCR) as follows:
RNA was dissolved in 10 µl RNAase-free water. cDNA was synthesized using 50 ng of the antisense primer ASI in a reaction mixture containing 2xTaq polymerase buffer (Promega Corp., Madison, WI) 0.5 mM dNTP, 20 units RNAsin (Promega), 10 mM dithiothreitol and 30 units avian myeloblastosis virus reverse transcriptase (Life Sciences, Bethesda, MD) for 60 min. at 42°C. PCR was performed in reaction mixture volume of 50 µl containing Taq Polymerase buffer (Promega), 2 mM dNTP, 1.5 mM MgCl₂, 20 ng of sense primer SI and 2.5 units Taq Polymerase (Promega). The reaction was carried out by 35 cycles of PCR consisting of 94°C for 1.5 min. 55°C for 1.5 min. and 72°C for 3 min. The second PCR reaction was performed as before, with 5 µl of the first PCR reaction mixture and the nested set of primers SII (sense) and ASII (antisense). The two sets of primers used are from the highly conserved 5' untranslated region (5' UTR).

The following primers were used:
*SI 7-26:* 5'-CAC-TCC-ACC-ATA-GAT-CAT-CCC-3'.
*ASI 248-222:* 5'-ACC-ACT-ACT-CGG-CTA-GCA-GT-3'
*SII 46-65:* 5'-TTC-ACG-CAG-AAA-GCG-TCT-AG-3'.
*ASII 190-171:* 5'-GTT-GAT-CCA-AGA-AAG-GAC-CC-3'.

The results obtained following transplantation of HCV infected liver fragments is shown in the following Table 3:

**Table 3 - Transplantation results of liver fragments from HCV infected human patients in SCID-BNX mice**

| **Number of Mice** | | | |
|---|---|---|---|
| **Transplanted with HCV infected live fragment** | **Surviving** | **Stably Engrafted** | **Viremia positive** |
| 20 | 17 | 15 | 8 |
| 17 | 14 | 10 | 7 |
| 13 | 11 | 6 | 5 |
| 12 | 10 | ND* | 6 |

| | | | |
|---|---|---|---|
| * ND = No Data: mice have not yet been sacrificed for histological examination | | | |

HCV sequences were first observed in the sera of the transplanted mice two weeks post transplantation and continued to bc detected intermittently for about two months after transplantation, at which time animals were sacrificed (a typical experiment is shown in Fig. 4). Similar fluctuations in detection of the HCV RNA have been previously observed in chimpanzees experimentally infected with HCV and in chronically infected patients, probably resulting from the very low levels of the virus in the serum.

### Example 5: Transplantation of Liver Fragments Infected in vitro with HCV to C3H Mice

C3H mice, which are not of an immune deficient strain, were irradiated by split total body irradiation (TBI) (a first dose of 400 rads and a second dose of 1,200 rads) and radioprotected the following day with 3x10⁶ SCID bone marrow cells (as described in Example 1 above).

Liver fragments were obtained from non HCV infected humans as described in Example 1 and the non infected human liver fragments were incubated *in vitro* with HCV resulting in their infection by HCV. The *in vitro* HCV infected liver fragments were transplanted under the kidney capsule of the C3H mice as described above and the detection of HCV in the transplanted mice was assessed by testing the level of HCV in the serum of the mice by RT-PCR as described in Example 4 above.

The results are shown in the following Table 4:

**Table 4 - Transplantation Results of in vitro Viral Hepatitis C Infected Human Livers in C3H Mice**

| **No. of Animals** | **No. of Animals Survived** | **No. of Animals Engrafted** | **Route of Infection** | **Infection Indicators and comments** |
|---|---|---|---|---|
| 10 | 10 | ND* | Pre-transplantation *in vitro* incubation of liver fragments with HCV-DNA, +HCV sera" | PCR +HCV 2/10 at day 14 |

| | | | | |
|---|---|---|---|---|
| * ND = No Data: Mice have not yet been sacrificed for histological examination ** +HCV sera = sera positive for HCV *** PCR +HCV = PCR test postitive for hepatitis C virus DNA. | | | | |

The results shown in the table above, demonstrate for the first time, that human liver fragments Infected *in vitro* with HCV may be engrafted and result in the infection of the transplanted mice with HCV as assessed by the detection of HCV sequences in the sera of the transplanted mice.

In addition, the above results, for the first time, show that human HCV infected livers may be transplanted under the kidney capsules of C3H mice, which are not of an immune deficient strain.

## Claims

1. A non-human chimeric animal useful as a model for human hepatitis virus (HV) infection, comprising a non-human mammal M5 having xenogeneic cells; mammal M5 being derived from a non-human mammal M1 treated to substantially destroy its hematopoietic cells and then transplanted with hematopoietic cells derived from one or more mammals M2 and transplanted with HBV- or HCV-infected liver tissue from a mammal M3, the one or more mammals M2 and mammal M3 being from the same or from a different species; the transplanted hematopoietic cells from the one or more mammals M2 being either one or both of a hematopoietic cell preparation from a T cell deficient mammal or of a T cell depleted mammalian stem cell or bone marrow preparation; the transplanted liver tissue from mammal M3 being either a human liver tissue preparation or a liver tissue preparation from a non-human mammal capable of being infected by HV.

2. A non-human animal according to Claim 1, wherein the hematopoietic cells of said mammal M1 are substantially destroyed by total body irradiation (TBI).

3. A non-human animal according to Claim 2, wherein the irradiation is a split total body irradiation (TBI).

4. A non-human animal according to Claims 1-3, wherein at least one of said mammals M2 is a T cell deficient mammal.

5. A non-human animal according to Claim 4, wherein the T cell deficient mammal M2 is a severe combined immuno-deficient (SCID) mammal.

6. A non-human animal according to Claim 5, wherein said mammal M2 is a SCID mouse.

7. A non-human animal according to any one of Claims 1 to 6, wherein the hematopoietic cell preparation is a T cell depleted bone-marrow preparation.

8. A non-human animal according to any one of Claims 1 to 7, wherein said stem cell preparation is a T cell depleted stem cell preparation obtained from peripheral blood of said M2 mammal.

9. A non-human animal according to Claim 8, wherein said M2 mammal is human undergoing treatment leading to migration of stem cells to the peripheral blood system.

10. A non-human animal according to Claim 9, wherein said treatment comprises administering of granulocyte colony stimulating factor (G-SCF) to said human.

11. A non-human animal according to any one of claims 1 to 10, wherein said M3 mammal is an HBV- or HCV-infected mammal.

12. A non-human animal according to Claim 11, wherein the HBV- or HCV-infected mammal is a human hepatitis patient.

13. A non-human animal according to any one of claims 1 to 10, wherein said M3 mammal is a non-HV infected mammal and the liver tissue is infected with HV in vitro prior to transplantation thereof into the M1 mammal.

14. A non-human animal according to any one of Claims 1 to 13, wherein the hematopoietic cells from the one or more mammals M2 comprise hematopoietic cells from a SCID mammal and a T cell depleted mammalian stem cell or bone marrow preparation.

15. A non-human animal according to claim 14, wherein the hemopoietic cells from the one or more mammals M2 comprise hematopoietic cells from a SCID mammal and a T cell depleted human stem cell or human bone marrow preparation.

16. A method for evaluating the potential of an agent or a combination of agents in the therapy or prevention of an HV infection using the non-human mammal M5, as defined in claim 1, comprising:
(a) administering said agent or said combination of agents to said mammal M5; and
(b) evaluating the effectiveness of said agent or said combination of agents in preventing spread of HV infection or reducing its physiological symptoms in said M5 mammal.

17. A method of obtaining anti-HV immune cells or antibodies said method comprising:
(a) recovering immune cells or antibodies from the blood of said M5 mammal; and
(b) selecting for the immune cells or antibodies having an anti-HV reactivity.

18. A method according to Claim 17, wherein the selected immune cells are anti-HV antibody producing B cells, the method further comprising a step of immortalizing said B cells.

19. A method according to claim 17, wherein the selected immune cells are cytotoxic T cells.

## Patentansprüche

1. Nicht-menschliches chimäres Tier nützlich als Modell für menschliche Hepatitsvirus(HV)-Infektion, umfassend einen nicht-menschlichen Säuger M5 mit xenogenen Zellen; wobei der Säuger M5 von einem nicht-menschlichen Säuger M1 abgeleitet ist, der behandelt wurde, um seine blutbildenden Zellen im Wesentlichen zu zerstören, und der dann mit blutbildenden Zellen abgeleitet von einem oder mehreren Säugern M2 und mit HBV- oder HCV-infiziertem Lebergewebe von einem Säuger M3 transplantiert wurde, wobei der/die Säuger M2 und der Säuger M3 von derselben oder einer unterschiedlichen Art sind; wobei die transplantierten blutbildenden Zellen von dem/den Säuger(n) M2, entweder aus einer blutbildenden Zellpräparation aus einem Säuger mit T-Zell-Mangel und/oder einer T-Zell-abgereicherten Stammzell- oder Knochenmarkspräparationen eines Säugers sind; wobei das transplantierte Lebergewebe von Säuger M3 entweder eine menschliche Lebergewebepräparation oder eine Lebergewebepräparation von einem nicht-menschlichen Säuger ist, der durch HV infiziert werden kann.

2. Nicht-menschliches Tier nach Anspruch 1, wobei die blutbildenden Zellen des Säugers M1 durch Ganzkörperbestrahlung (TBI) im Wesentlichen zerstört sind.

3. Nicht-menschliches Tier nach Anspruch 2, wobei die Strahlung eine geteilte Ganzkörperbestrahlung (TBI) ist.

4. Nicht-menschliches Tier nach den Ansprüchen 1 bis 3, wobei mindestens einer der Säuger M2 ein Säuger mit T-Zell-Mangel ist.

5. Nicht-menschliches Tier nach Anspruch 4, wobei der Säuger M2 mit T-Zell-Mangel ein Säuger mit schwerem kombiniertem Immundefekt (SCID) ist.

6. Nicht-menschliches Tier nach Anspruch 5, wobei der Säuger M2 eine SCID-Maus ist.

7. Nicht-menschliches Tier nach einem der Ansprüche 1 bis 6, wobei die blutbildende Zellpräparation eine von T-Zellen abgereicherte Knochenmarkspräparation ist.

8. Nicht-menschliches Tier nach einem der Ansprüche 1 bis 7, wobei die Stammzellpräparation von T-Zellen abgereichert ist und aus dem peripherem Blut des Säugers M2 erhalten wurde.

9. Nicht-menschliches Tier nach Anspruch 8, wobei der M2-Säuger menschlich ist und sich einer Behandlung unterzieht, die zur Migration von Stammzellen ins periphere Blutsystem führt.

10. Nicht-menschliches Tier nach Anspruch 9, wobei die Behandlung die Verabreichung von Granulozyten-Kolonie-stimulierendem Faktor (G-CSF) an den Menschen umfasst.

11. Nicht-menschliches Tier nach einem der Ansprüche 1 bis 10, wobei der M3-Säuger ein HBV- oder HCV-infizierter Säuger ist.

12. Nicht-menschliches Tier nach Anspruch 11, wobei der HBV- oder HCVinfizierte Säuger ein menschlicher Hepatitispatient ist.

13. Nicht-menschliches Tier nach einem der Ansprüche 1 bis 10, wobei der M3-Säuger ein nicht HV-infizierter Säuger ist und wobei das Lebergewebe vor seiner Transplantation in den M1-Säuger *in vitro* mit HV infiziert wurde.

14. Nicht-menschliches Tier nach einem der Ansprüche 1 bis 13, wobei die blutbildenden Zellen von dem/den Säuger(n) M2 blutbildende Zellen eines SCID-Säugers und eine von T-Zellen abgereicherte Stammzell- oder Knochenmarkspräparation aus Säugern umfassen.

15. Nicht-menschliches Tier nach Anspruch 14, wobei die blutbildenden Zellen von dem/den Säuger(n) M2 blutbildende Zellen eines SCID-Säugers und eine von T-Zellen abgereicherte menschliche Stammzell- oder Knochenmarkspräparation enthalten.

16. Verfahren zur Abschätzung des Potentials eines Agens oder einer Kombination von Agenzien in der Therapie oder Vorbeugung einer HV-Infektion unter Verwendung des nicht-menschlichen Säugers M5, wie in Anspruch 1 definiert, wobei das Verfahren umfasst:
a) Verabreichen des Agens oder der Kombination von Agenzien an den Säuger M5; und
b) Abschätzen der Wirksamkeit des Agens oder der Kombination von Agenzien beim Verhindern der Ausbreitung von HV-Infektion oder beim Verringern ihrer physiologischen Symptome in dem M5-Säuger.

17. Verfahren zum Erhalten von Anti-HV-Immunzellen oder Antikörpern unter Verwendung des nicht-menschlichen Säugers M5, wie in Anspruch 1 definiert, wobei das Verfahren umfasst:
a) Erhalten von Immunzellen oder Antikörpern aus dem Blut des M5-Säugers; und
b) Auswählen von Immunzellen oder Antikörpern mit einer Anti-HV-Reaktivität.

18. Verfahren nach Anspruch 17, wobei die ausgewählten lmmunzellen Anti-HV-Antikörper-produzierende B-Zellen sind und das Verfahren ferner einen Schritt der Immortalisierung der B-Zellen umfasst.

19. Verfahren nach Anspruch 17, wobei die ausgewählten Immunzellen cytotoxische T-Zellen sind.

## Revendications

1. Animal chimère non humain utile comme modèle pour l'infection par le virus humain de l'hépatite (VH), comprenant un mammifère non humain M5 présentant des cellules xénogéniques ; le mammifère M5 étant dérivé d'un mammifère non humain M1 traité pour substantiellement détruire ses cellules hématopoïétiques puis transplanté avec des cellules hématopoïétiques dérivées d'un ou plusieurs mammifère(s) M2 et transplanté avec du tissu hépatique infecté par VHB ou VHC d'un mammifère M3, le ou les mammifère(s) M2 et le mammifère M3 provenant de la même espèce ou d'espèces différentes ; les cellules hématopoïétiques transplantées du ou des mammifère(s) M2 étant soit ou à la fois une préparation à base de cellules hématopoïétiques d'un mammifère présentant une déficience en lymphocytes T ou une préparation à base de moelle osseuse ou de cellules souches de mammifère déplétée en lymphocytes T ; le tissu hépatique transplanté du mammifère M3 étant soit une préparation de tissu hépatique humain soit une préparation de tissu hépatique d'un mammifère non humain susceptible d'être infecté par VH.

2. Animal non humain selon la revendication 1, dans lequel les cellules hématopoïétiques dudit mammifère M1 sont substantiellement détruites par irradiation corporelle totale (ICT).

3. Animal non humain selon la revendication 2, dans lequel l'irradiation est une irradiation corporelle totale (ICT) fractionnée.

4. Animal non humain selon les revendications 1 à 3, dans lequel au moins un desdits mammifères M2 est un mammifère présentant une déficience en lymphocytes T.

5. Animal non humain selon la revendication 4, dans lequel le mammifère M2 présentant une déficience en lymphocytes T est un mammifère atteint d'un déficit immunitaire combiné sévère (DICS).

6. Animal non humain selon la revendication 5, dans lequel ledit mammifère M2 est une souris DICS.

7. Animal non humain selon l'une quelconque des revendications 1 à 6, dans lequel la préparation de cellules hématopoïétiques est une préparation de moelle osseuse déplétée en lymphocytes T.

8. Animal non humain selon l'une quelconque des revendications 1 à 7, dans lequel ladite préparation de cellules souches est une préparation de cellules souches déplétée en lymphocytes T obtenue du sang périphérique dudit mammifère M2.

9. Animal non humain selon la revendication 8, dans lequel ledit mammifère M2 est un humain subissant un traitement conduisant à la migration de cellules souches vers le système sanguin périphérique.

10. Animal non humain selon la revendication 9, dans lequel ledit traitement comprend l'administration de facteur de stimulation des colonies de granulocyte (G-CSF) audit humain.

11. Animal non humain selon l'une quelconque des revendications 1 à 10, dans lequel ledit mammifère M3 est un mammifère infecté par VHB ou VHC.

12. Animal non humain selon la revendication 11, dans lequel le mammifère infecté par VHB ou VHC est un patient humain atteint d'une hépatite.

13. Animal non humain selon l'une quelconque des revendications 1 à 10, dans lequel ledit mammifère M3 est un mammifère non infecté par VH et dans lequel le tissu hépatique est infecté avec VH in vitro préalablement à la transplantation de celui-ci dans le mammifère M1.

14. Animal non humain selon l'une quelconque des revendications 1 à 13, dans lequel les cellules hématopoïétiques du ou des mammifère(s) M2 comprennent des cellules hématopoïétiques d'un mammifère DICS et une préparation de moelle osseuse ou de cellules souches de mammifère déplétée en lymphocytes T.

15. Animal non humain selon la revendication 14, dans lequel les cellules hématopoïétiques du ou des mammifère(s) M2 comprennent des cellules hématopoïétiques d'un mammifère DICS et une préparation de moelle osseuse humaine ou de cellules souches humaines déplétée en lymphocytes T.

16. Procédé permettant d'évaluer le potentiel d'un agent ou d'une combinaison d'agents dans le traitement ou la prévention d'une infection par VH en utilisant le mammifère non humain M5, tel que défini dans la revendication 1, ledit procédé comprenant :
(a) l'administration dudit agent ou de ladite combinaison d'agents audit mammifère M5 ; et
(b) l'évaluation de l'efficacité dudit agent ou de ladite combinaison d'agents à prévenir la propagation de l'infection par VH ou à réduire ses symptômes physiologiques dans ledit mammifère M5.

17. Procédé permettant d'obtenir des cellules immunitaires ou des anticorps anti-VH en utilisant le mammifère non humain M5, tel que défini dans la revendication 1, ledit procédé comprenant :
(a) la récupération de cellules immunitaires ou d'anticorps du sang dudit mammifère M5 ; et
(b) la sélection des cellules immunitaires ou des anticorps ayant une réactivité anti-VH.

18. Procédé selon la revendication 17, dans lequel les cellules immunitaires sélectionnées sont des cellules B produisant des anticorps anti-VH, le procédé comprenant en outre une étape consistant à immortaliser lesdites cellules B.

19. Procédé selon la revendication 17, dans lequel les cellules immunitaires sélectionnées sont des cellules T cytotoxiques.
